# EUROPEAN PATENT APPLICATION

(11) **EP 4 497 368 A1**
(43) Date of publication of application: **29.01.2025**
(21) Application number: 24158203.0
(22) Date of filing: 16.02.2024
(51) Int. Cl.: A61B 1/00, A61B 1/267

(54) **ENDOSCOPIC IMAGING MANIPULATION METHOD AND SYSTEM**

(30) Priority: 25.07.2023 US 202363528705 P
(71) Applicant: Olympus Medical Systems Corporation, Hachioji-shi Tokyo 192-8507 (JP)
(72) Inventor: LAZZARI, Matteo, 22303 Hamburg (IT); ARIYOSHI, Daiki, Chofu City (JP); SAKAI, Yuji, Kodaira-city, 187-0011 (JP)
(74) Representative: Seemann & Partner Patentanwälte mbB

(57) **Abstract**

The present disclosure concerns an endoscopic imaging manipulation method and system. The method comprises capturing endoscopic images of laryngeal and/or pharyngeal tissue structures during examination of the larynx and/or pharynx using an endoscope inserted through a patient's nose or mouth, feeding the captured endoscopic images to an instance of an artificial intelligence trained to identify suspicious areas of laryngeal and/or pharyngeal tissue structures showing signs of alterations from healthy laryngeal and/or pharyngeal tissue, overlaying the captured endoscopic images with a marking indicating areas indicated by the instance of artificial intelligence as suspicious, and displaying the overlayed captured endoscopic images on a monitor.

## Description

The present disclosure relates to an endoscopic imaging manipulation method and system.

Laryngeal and pharyngeal biopsies are a routine diagnostic procedure in ENT (ear, nose, throat) medicine to evaluate and examine the larynx and other parts of the upper aerodigestive tract. The procedure is performed using a flexible or rigid endoscope inserted through the patient's nose or mouth. This allows the visualization of the pharynx and the larynx and identification of any suspicious areas or changes in the mucosal layer that may indicate inflammation or cancer.

Upon identification of a suspicious area in the pharynx or larynx, a biopsy from the suspicious area may be conducted by removing a small tissue sample for examination. Historically, such biopsies have been performed under general anesthesia using a microscope. However, this approach is associated with significant costs, including the utilization of operating room staff and general anesthesia, as well as the potential for complications due to the use of general anesthesia, particularly in patients with comorbidities. Additionally, the increased waiting lists resulting from the pandemic may lead to delays in the biopsy procedure, potentially impacting diagnostic accuracy and patient outcomes.

An overarching primary objective of the above-described procedures is to preserve the anatomy, thus ensuring good patient outcomes, including voice and functional preservation. These objectives are achieved through less invasive biopsy techniques.

In this respect, an alternative approach to biopsy of the larynx is the use of local anesthesia, often performed immediately following diagnostic laryngoscopy, in an office setting. This variant of the technique involves the introduction of a flexible endoscope, which may be reusable or single use, through the nose. Once a suspicious area is identified, a small forceps is inserted through a channel of the endoscope to obtain a tissue sample. This procedure takes only a few minutes and has been shown to be as safe and efficient as the traditional method while improving patient outcomes and diagnostic accuracy and reducing overall hospital costs.

However, this technique may present certain challenges during execution. As the patient is awake during the procedure, the anatomy may move and reflexes may occur, which would potentially affect the success of the biopsy and necessitate multiple attempts to obtain an adequate tissue sample. Additionally, the patient may experience discomfort during the procedure.

These circumstances act against the recommendations of guidelines recommend to preserve as much tissue as possible to maintain voice and functionality.

One of the main challenges in performing biopsies under local anesthesia is the precise identification of the lesion's core. Determining the anatomic location that is most likely to yield the most accurate histological results is essential for the success of the procedure.

To achieve this, training and experience are essential. However, trained and experienced physicians may be in short supply, particularly in rural areas. In conclusion, in the mentioned area and cases, the traditional method of performing biopsies under general anesthesia will continue to be used, with the previously explained limitations.

In light of this, there is a need for solutions for more ENT physicians to be enabled to perform laryngeal biopsies under local anesthesia.

This need is met with an endoscopic imaging manipulation method, the method comprising:
capturing endoscopic images of laryngeal and/or pharyngeal tissue structures during examination of the larynx and/or pharynx using an endoscope inserted through a patient's nose or mouth,
feeding the captured endoscopic images to an instance of an artificial intelligence trained to identify suspicious areas of laryngeal and/or pharyngeal tissue structures showing signs of alterations from healthy laryngeal and/or pharyngeal tissue,
overlaying the captured endoscopic images with a marking indicating areas indicated by the instance of artificial intelligence as suspicious, and
displaying the overlayed captured endoscopic images on a monitor.

Numerous clinical studies have shown a correlation between different vascular patterns and various disease states. Alterations in blood vessel morphology and density have been shown to reflect the severity of the disease. At this moment, four different vascular classifications that are based on NBI have been widely adopted in the field of otolaryngology. These classifications aid in the visual identification of the type of lesion and disease. The presently disclosed method and system are directed to enhancing the diagnostic capabilities of otolaryngology physicians by incorporating an instance of artificial intelligence (AI) and machine learning (ML) techniques in conjunction with NBI, to guide targeted biopsy procedures and thereby improve patient outcomes.

Such an overlay, to be displayed for example within a specific GUI (Graphical user interface) will identify the lesion and the margin delimitation with higher accuracy rate, since small vessels that might be part of a lesion are often not visible to the human eye. If missed, these can lead to recurrences. Together with the fact that NBI images are used, physicians in otolaryngology are aided by the overlay, even if they are less experienced, e.g., for a lack of cases in less populated areas.

In embodiments, the endoscopic images are endoscopic NBI images. Narrow band imaging (NBI) is a medical imaging technique that utilizes specific wavelengths of light to enhance the visualization of blood vessels, mucosa and other structures. This technology is widely used in the field of ENT (Ear, Nose, and Throat) to improve the visualization of vessels.

In an embodiment, the instance of an artificial intelligence being may be a convolutional neural network (CNN) having a classifier, the CNN having been trained by at least one of supervised and unsupervised learning of a multitude of endoscopic images of laryngeal and/or pharyngeal structures to classify suspicious areas of laryngeal and/or pharyngeal tissue in the captured endoscopic images. Thereby, using pre-set training data, images and videos, as well as the system's capability to learn, the system can be trained to classify suspicious areas in NBI imagery of the larynx.

The term unsupervised learning typically describes a process wherein the neural network is trained using only images of healthy laryngeal and/or pharyngeal tissue. Any region that does not conform to what the neural network as learned has being healthy will be marked suspicious. Supervised training takes the training a step further. Diseased areas are marked and classified beforehand in the training data so that the neural network is trained to not only identify suspicious areas, but also provide an estimate about what kind of lesion is shown in an NBI image.

In embodiments, the instance of an artificial intelligence may have been trained to identify alterations from healthy laryngeal and/or pharyngeal tissue as suspicious stemming from one or more of lesions, blood vessel morphology, blood vessel density, vascular patterns, and structure of the mucosal surface. By this, the disclosed algorithm utilizes image analysis techniques to aid otolaryngology physicians in the identification of vascular patterns, density, morphology, and structure on the mucosal surface. By providing such information, the algorithm aims to support the diagnostic capabilities of the physician and assist in the identification of potential pathology.

In further embodiments, the overlay may be created as a color-coded or brightness-code heat map. In particular, the color-coding or brightness-coding of the heat map may, in further embodiments, be based on at least one of the density and pattern of vessels in suspicious areas.

A color coded (heat) map overlay based on the density and pattern of vessels enhances targeted biopsy and decreases the likelihood of false results or unnecessary invasive procedures. Thicker and irregular areas may be represented with darker colors, while regular and normal vessels will be represented with lighter, for example greener, colors. The color scheme may be in accordance with established classifications.

Physicians will be able to view a thermal map overlay on the designated lesion. The AI instance will guide physicians to perform the biopsy in the darkest area of the map, which represents the core of the lesion, where the histological results are likely to indicate the highest degree of disease.

The instance of artificial intelligence may be setup to learn from new endoscopic images that are captured during subsequent examinations of the larynx and/or pharynx, after initial training has been completed, in embodiments, .

The captured endoscopic images may be displayed on a monitor without overlay upon request, thus allowing the physician to inspect a suspicious area unobstructed by overlays. With this, the AI instance may be activated or deactivated during a procedure, dependent on the physician's needs at any given time during the procedure.

The object is also achieved by an endoscopic imaging manipulation system comprising a video endoscope suited to be fed through a patient's mouth or nose for laryngeal examination, a light source capable of providing white light for white light imaging (WLI) and narrow band lighting for narrow band imaging (NBI) connected to or integrated into the video endoscope, an image analyzer connected to the video endoscope for receiving endoscopic images from the endoscope, the image analyzer having an instance of an artificial intelligence trained to identify suspicious areas of laryngeal and/or pharyngeal tissue structures showing signs of alterations from healthy laryngeal and/or pharyngeal tissue, the image analyzer further being configured to overlay, the captured endoscopic images with a marking indicating areas indicated by the instance of artificial intelligence as suspicious, and a monitor connected to the image analyzer for displaying endoscopic images provided by the image analyzer.

The system embodies the same features and advantages as the endoscopic imaging manipulation method.

In embodiments, the image analyzer may be configured to apply the instance of artificial intelligence to the captured endoscopic images when NBI is applied, based on one of an imaging mode identification signal or image characteristics in the captured images indicative of NBI.

In a further embodiment, the image analyzer is configured to apply the instance of artificial intelligence to the captured endoscopic images upon request by a practitioner.

The instance of an artificial intelligence may in an embodiment be a convolutional neural network (CNN) having a classifier, the CNN having been trained by at least one of supervised and unsupervised learning of a multitude of endoscopic images of laryngeal and/or pharyngeal structures to classify suspicious areas of laryngeal and/or pharyngeal tissue in the captured endoscopic images.

In another embodiment, the instance of an artificial intelligence has been trained to identify alterations from healthy laryngeal and/or pharyngeal tissue as suspicious stemming from one or more of lesions, blood vessel morphology, blood vessel density, vascular patterns, and structure of the mucosal surface.

In embodiments, the image analyzer may be configured to create the overlay as a color-coded or brightness-code heat map, wherein in particular the image analyzer may be configured to base the color-coding or brightness-coding of the heat map on at least one of the density and pattern of vessels in suspicious areas.

A GUI may be configured with user-customized colors, shapes and other options to display estimated shape, size, classification and confidence rating for identified lesion(s). A lesion map delineating the parameters of mucosal changes may be displayed as well, possibly accompanied with written information about size and type, accompanied by associated accuracy ratings. The GUI may also display patient information, imaging modes such as WLI (white light imaging) and NBI, and activation status of the system, in particular the AI instance.

Further information that may be displayed using the GUI may be, based on the thermal map area, shape and size, an automated recommendation of an appropriate number of biopsies to ensure optimal sampling and comprehensive mapping of all suspicious regions, or, based on an identification of the endoscope connected to the system through an ID chip embedded in the endoscope, an automated recommendation and suggestion of compatible accessories to prevent potential damage to the endoscope resulting from an incorrect instrument being used. The GUI may also be recorded for later study or analysis.

The instance of artificial intelligence can be setup to learn from new endoscopic mages in embodiments that are captured during subsequent examinations of the larynx and/or pharynx, after initial training has been completed.

In order keep on learning, the image analyzer may be configured to receive feedback from a practitioner carrying out a laryngeal examination about the unmarked suspicious areas in the laryngeal and/or pharyngeal tissue structures or the lack thereof and/or about whether the classification of one or more suspicious areas of laryngeal and/or pharyngeal tissue structures is correct or not.

In embodiments, the system may reconstruct the information into a 2D lesion map, providing a clearer representation of the anatomical area in question and the location. This will aid doctors in better identifying and mapping suspicious areas.

Further characteristics of the invention will become apparent from the description of the embodiments according to the invention together with the claims and the included drawings. Embodiments according to the invention can fulfill individual characteristics or a combination of several characteristics.

The invention is described below, without restricting the general intent of the invention, based on exemplary embodiments, wherein reference is made expressly to the drawings with regard to the disclosure of all details according to the invention that are not explained in greater detail in the text. The drawings show in:
- Fig. 1: a schematic representation of the steps of the image manipulation method,
- Fig. 2: a schematic representation of an embodiment of an instance of an artificial intelligence,
- Fig. 3: an embodiment of a GUI representing the result of the present method,
- Fig. 4: representations of training data for the instance of artificial intelligence and
- Fig. 5: a schematic representation of a system according to the present disclosure.

In the drawings, the same or similar types of elements or respectively corresponding parts are provided with the same reference numbers in order to prevent the item from needing to be reintroduced.

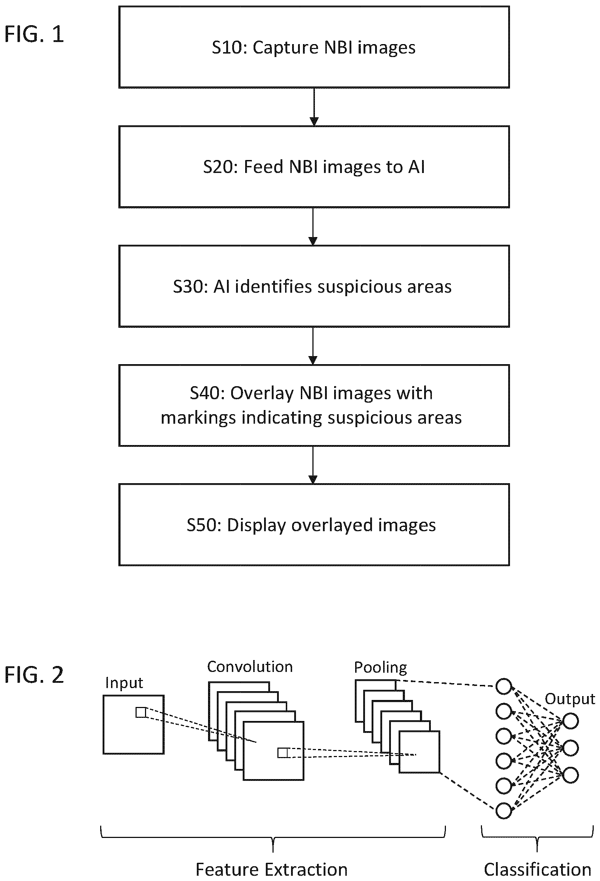

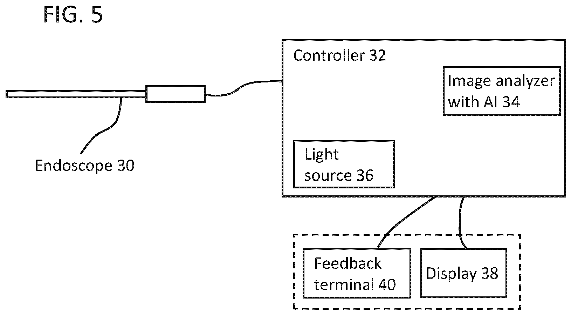

Fig. 1 displays a schematic representation of the steps of the image manipulation method, which can be carried out using a system, an exemplary embodiment of which is shown Fig. 5. In step S10, narrowband imaging (NBI) images of the larynx of the patient are captured using a video endoscope, which may have an image sensor at its distal end, or maybe of a different type with an image sensor at the proximal end or a separate camera head attached to a purely optical endoscope. These are sent to an image processor in step S20 that runs an instance of artificial intelligence that has been trained with NBI images of the larynx to identify suspicious areas in the mucous surface or the tissue of the larynx. In step S30, the instance of artificial intelligence processes the NBI images and identifies suspicious areas according to its training. The identification of suspicious areas can be done by classifying such areas as suspicious, or, in the case of enhanced sophistication, by classifying different types of suspicious areas according to the underlying disease and/or lesion severity.

The instance of artificial intelligence provides the suspicious areas and that classification as outputs, so that in step S40, the image analyzer is able to overlay the NBI images with markings indicating the suspicious areas found by the instance of artificial intelligence. The overlaid images are then displayed to personnel conducting an examination or a biopsy on a monitor in step S50.

Fig. 2 shows a schematic representation of an embodiment of an instance of an artificial intelligence useful for the identification of suspicious areas in NBI images of the larynx. This embodiment of an instance of artificial intelligences is configured as a convolutional neural network (CNN) having a classifier on the output side. The NBI images are fed into the CNN on the input side and undergo a feature extraction by being processed inside the CNN's convolutional base ("convolution") and pooled ("pooling") according to the known principles of working of convolutional neural networks. The convolution and putting stages may be repeated once or a few times. As a result of the feature extraction, features of the NBI images that may indicate suspicious areas are identified. These are then set into the classification layer of the CNN, which may be a fully connected layer, or several layers, and processed towards an output layer, in which each node represents a classification that the CNN has been trained for. In the simplest case, there may be only a very few nodes in the output layer that represent the presence or absence of a suspicious area. In more sophisticated embodiments, the output layer may have several nodes representing different diseases, lesion types, severities, etc.

The explanation of the principle using a convolutional neural network is not to be construed as limiting, as other examples of artificial intelligence and machine learning may also be trained and employed for this purpose, such as, e.g., support vector machines, learning vector quantization, or random forest models.

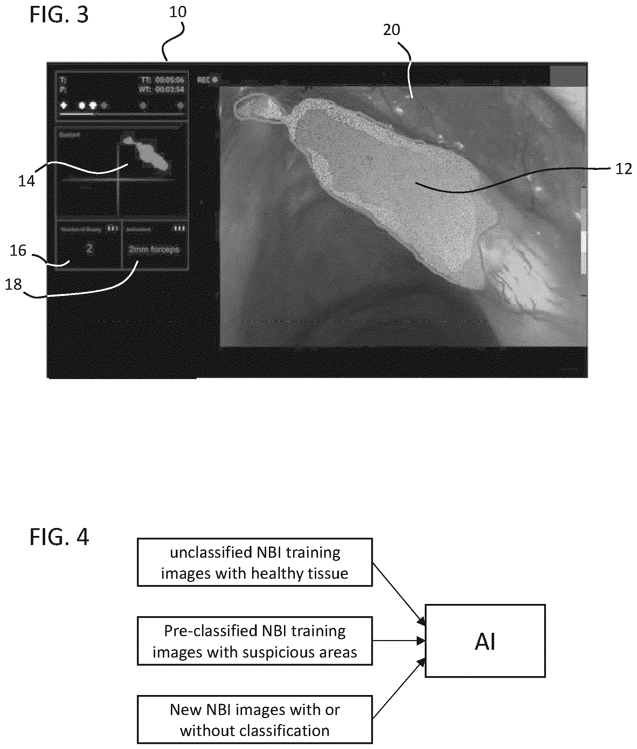

Fig. 3 represents an embodiment of a GUI 10 representing the result of the present method. The GUI 10 will be shown on a monitor to a physician performing an endoscopic examination and possibly biopsy of the larynx of the patient and is divided into several partial screens displayed next to each other. The large partial screen on the right displays the NBI image of the larynx as currently captured by an endoscope, with healthy laryngeal and/or pharyngeal tissue 20 shown in an unaltered manner. Since the NBI image has been processed by an instance of artificial intelligence trained for this purpose, a disease or lesioned area has been classified as being suspicious and is indicated inside the image as an overlay 12 in the form of a heat map. The heat map overlay 12 has different colors indicating the level of confidence in the classification, resulting in the center of the suspicious area being displayed in a darker shade than its surrounding area. As can be seen in Fig. 3, a boundary line is additionally drawn around the suspicious area.

In the left part of GUI 10, several inserts are displayed that provide information about the suspicious area as well as about suggestions that are based on the findings of the instance of artificial intelligence about the suspicious area. The uppermost insert may contain patient information as well as the duration of the procedure and an indication of the progress of the examination in the form of a progress bar, above which are displayed symbols signifying anatomical milestones that have been passed during the examination, such as the mouth (diamond shaped symbol), the tonsils (circular symbol) and the larynx (shell shaped symbol). These symbols are highlighted because the endoscope tip has passed or reached these milestones. Later milestones on the way to the lung have not been reached and are still grayed out.

Below the first insert, a second insert shows the outline of the suspicious area relative to its position inside the throat. As can be seen from the second insert, the suspicious area is located in the upper right part of the throat. Its relative size with respect to the third can also be derived from this graphic representation.

The third insert contains two pieces of information, namely a number ("2"), the number being a suggestion for the number of biopsies to be taken from the specific suspicious area on display, whereas the second information is a suggestion of a type of forceps to be used for the biopsies. A forceps can be introduced through a channel in the endoscope, so that the physician carrying out the examination and biopsy will be able to see the handling of the forceps while she is performing the biopsy. The physician may have the ability to cause the image analyzer to stop displaying the overlay 12 in order to be able to observe the lesioned or diseased tissue to be biopsied, or to change the transparency of the overlay. In another embodiment, only the outline of the suspicious area will be retained in the image. The representation 14 on the left side may be retained including the various colors or shades of the heat map so that the physician will have simultaneously a clear view of the area she is operating on and the heat map representing the classification, which may indicate the most promising locations to take tissue samples through either specific coloring or shading or through distinctive markings at the location of the suggested locations. Such markings, if they are visible, but unobtrusive, may be kept in the large image when the rest of the heat map is made transparent or removed from the image for the purpose of the biopsy.

The marking of a suspicious area that has been identified by the instance of artificial intelligence in NBI images may remain in the display after the imaging has been switched over to white light imaging (WLI), with some lateral movement and magnification is a shift of the location of the endoscope is detected in the images. This is in particular helpful for the actual act of taking biopsy samples from the suspicious area.

Fig. 4 shows exemplary representations of training data for the instance of artificial intelligence. The uppermost training data are unclassified NBI training images that contain only healthy tissue. Such training data are needed for so-called unsupervised training of the artificial intelligence. The training will prompt the instance of the artificial intelligence to accept any laryngeal and/or pharyngeal tissues and services as on suspicious that conform to these training data. If there is a deviation from the structures that have been trained as being normal, the affected area will show up automatically and be classified as a suspicious area.

The alternative case of so-called supervised learning is based on free classified and be a training images, some of which only display healthy tissue, but others of which contain lesioned or diseased areas, in which such affected areas are indicated and classified, either simply as suspicious or, in higher granularity, regarding their types and severities. The supervised training results in an optimization such that specific nodes in the output layer of a CNN are trained to indicate the various types and severities, respectively.

The third kind of input images indicated in Fig. 4 are new endoscopic NBI images that are captured during examinations and biopsies performed using the pre-trained instance of artificial intelligence and are used to continuously update the training of the artificial intelligence instance, both in unsupervised and in supervised learning. In the case of unsupervised learning, the physician performing an examination may indicate that the present view of the larynx is free from suspicious areas. In that case, the NBI image can be used for further establishing the ground truth of NBI training images for unsupervised learning. Alternatively, in case of the presence of suspicious areas of the physician may confirm the finding and classification of a suspicious area or alter a finding that he has found to be incorrect, so that this image including its correct classification can be used for further supervised training of the instance of artificial intelligence.

Fig. 5 is a schematic representation of a system according to the present disclosure. The system comprises an endoscope 30, which may be a flexible endoscope having an optics and an image sensor at its distal end, or a rigid endoscope with an internal relay optics and an image sensor in its handle or in a separate camera head that is attached to the endoscope. The image sensor is connected to a controller 32 of the system used to control the imaging, lighting and other parameters of an endoscopic examination. The controller 32 may comprise a light source 36 capable of producing white light for WLI and alternative the narrowband lighting for NBI. A light source may also be implemented in the endoscope 30 and controlled by controller 32. The controller 32 furthermore comprises an image analyzer 34 running an instance of artificial intelligence 34 trained to identify suspicious areas in laryngeal and/or pharyngeal tissue according to the previous description. The image analyzer 34 is also configured to provide the NBI images with overlays that visualize or highlight suspicious areas detected by the instance of artificial intelligence. Examples of such overlays are shown and described with respect to Fig. 3, for example.

The overlaid images are displayed using a monitor or display 38, for example in the form of a GUI 10 as shown in Fig. 3, possibly including further information, such as information about the size, type and/or severity of suspicious areas, or suggestions for the number and/or locations of biopsy samples to be taken or about the type of forceps to be used.

Furthermore the system may include a feedback terminal 40, providing a physician carrying out an examination of the larynx to provide feedback about the findings of the instance of artificial intelligence with respect to suspicious areas, for example confirming or altering such findings. Such feedback may be used for further training of the instance of artificial intelligence, as explained with respect to Fig. 4. Such feedback terminal 14 may be a separate device, such as a computer, or be integrated into the display, as indicated by the dashed line around display 38 and feedback terminal 40, in which case the display has a touchscreen and the GUI 10 displays defined areas of the screen for feedback by touching the touch screen. For example, a touch on an insert area of GUI 10 displaying information about type or severity of a lesion or otherwise suspicious area may open a dialogue for correcting the automatically generated information with the physician's findings. In place of input by touchscreen, the correction may also carried out via voice recognition or other suitable means.

All named characteristics, including those taken from the drawings alone, and individual characteristics, which are disclosed in combination with other characteristics, are considered alone and in combination as important to the invention. Embodiments according to the invention can be fulfilled through individual characteristics or a combination of several characteristics. Features which are combined with the wording "in particular" or "especially" are to be treated as preferred embodiments.

### List of References

- 10: GUI
- 12: heat map overlay
- 14: representation of suspicious area
- 16: suggestion for number of biopsy samples
- 18: suggestion for forceps to be used
- 20: laryngeal and/or pharyngeal tissue
- 30: endoscope
- 32: controller
- 34: image analyzer with instance of AI
- 36: light source
- 38: display
- 40: feedback terminal

## Claims

1. Endoscopic imaging manipulation method, the method comprising:
Capturing endoscopic images of laryngeal and/or pharyngeal tissue structures during examination of the larynx and/or pharynx using an endoscope inserted through a patient's nose or mouth,
Feeding the captured endoscopic images to an instance of an artificial intelligence trained to identify suspicious areas of laryngeal and/or pharyngeal tissue structures showing signs of alterations from healthy laryngeal and/or pharyngeal tissue,
Overlaying the captured endoscopic images with a marking indicating areas indicated by the instance of artificial intelligence as suspicious, and
Displaying the overlayed captured endoscopic images on a monitor.

2. The endoscopic imaging manipulation method of claim 1, wherein the endoscopic images being endoscopic NBI images.

3. The endoscopic imaging manipulation method of claim 1 or 2, wherein the instance of an artificial intelligence being a convolutional neural network (CNN) having a classifier, the CNN having been trained by at least one of supervised and unsupervised learning of a multitude of endoscopic images of laryngeal and/or pharyngeal structures to classify suspicious areas of laryngeal and/or pharyngeal tissue in the captured endoscopic images.

4. The endoscopic imaging manipulation method of claim 3, wherein the instance of artificial intelligence being setup to learn from new endoscopic images that are captured during subsequent examinations of the larynx and/or pharynx, after initial training has been completed.

5. The endoscopic imaging manipulation method of one of claims 1 to 4, wherein the instance of an artificial intelligence having been trained to identify alterations from healthy laryngeal and/or pharyngeal tissue as suspicious stemming from one or more of lesions, blood vessel morphology, blood vessel density, vascular patterns, and structure of the mucosal surface.

6. The endoscopic imaging manipulation method of one of claims 1 to 5, wherein the overlay being created as a color-coded or brightness-code heat map, wherein in particular the color-coding or brightness-coding of the heat map being based on at least one of the density and pattern of vessels in suspicious areas.

7. The endoscopic imaging manipulation method of one of claims 1 to 6, wherein the captured endoscopic images are displayed on a monitor without overlay upon request.

8. Endoscopic imaging manipulation system comprising a video endoscope suited to be fed through a patient's mouth or nose for laryngeal examination, a light source capable of providing white light and narrow band lighting for white light imaging (WLI) and narrow band imaging (NBI) connected to or integrated into the video endoscope, an image analyzer connected to the video endoscope for receiving endoscopic images from the endoscope, the image analyzer having an instance of an artificial intelligence trained to identify suspicious areas of laryngeal and/or pharyngeal tissue structures showing signs of alterations from healthy laryngeal and/or pharyngeal tissue, the image analyzer further being configured to overlay, the captured endoscopic images with a marking indicating areas indicated by the instance of artificial intelligence as suspicious, and a monitor connected to the image analyzer for displaying endoscopic images provided by the image analyzer.

9. The endoscopic imaging manipulation system of claim 8, wherein the image analyzer being configured to apply the instance of artificial intelligence to the captured endoscopic images when NBI is applied, based on one of an imaging mode identification signal or image characteristics in the captured images indicative of NBI.

10. The endoscopic imaging manipulation system of claim 8 or 9, wherein the image analyzer being configured to apply the instance of artificial intelligence to the captured endoscopic images upon request by a practitioner.

11. The endoscopic imaging manipulation system of one of claims 8 to 10, wherein the instance of an artificial intelligence being a convolutional neural network (CNN) having a classifier, the CNN having been trained by at least one of supervised and unsupervised learning of a multitude of endoscopic images of laryngeal and/or pharyngeal structures to classify suspicious areas of laryngeal and/or pharyngeal tissue in the captured endoscopic images.

12. The endoscopic imaging manipulation system of one of claims 8 to 11, wherein the instance of an artificial intelligence having been trained to identify alterations from healthy laryngeal and/or pharyngeal tissue as suspicious stemming from one or more of lesions, blood vessel morphology, blood vessel density, vascular patterns, and structure of the mucosal surface.

13. The endoscopic imaging manipulation system of one of claims 8 to 12, wherein the image analyzer being configured to create the overlay as a color-coded or brightness-code heat map, wherein in particular the image analyzer being configured to base the color-coding or brightness-coding of the heat map on at least one of the density and pattern of vessels in suspicious areas.

14. The endoscopic imaging manipulation system of one of claims 8 to 13, wherein the instance of artificial intelligence being setup to learn from new endoscopic images that are captured during subsequent examinations of the larynx and/or pharynx, after initial training has been completed.

15. The endoscopic imaging manipulation system of claim 14, wherein the image analyzer is configured to receive feedback from a practitioner carrying out a laryngeal examination about the unmarked suspicious areas in the laryngeal and/or pharyngeal tissue structures or the lack thereof and/or about whether the classification of one or more suspicious areas of laryngeal and/or pharyngeal tissue structures is correct or not.
